# EUROPEAN PATENT APPLICATION

(11) **EP 2 093 566 A1**
(43) Date of publication of application: **26.08.2009**
(21) Application number: 08380052.4
(22) Date of filing: 21.02.2008
(51) Int. Cl.: G01N 33/574, C12Q 1/68

(54) **Method for the prognosis of non-small cell lung cancer**

(71) Applicant: Pangaea Biotech, S.A., 08028 Barcelona (ES)
(72) Inventor: Tarón Roca, Miguel, 08916 Badalona (ES); Rosell Costa, Rafael, 08916 Badalona (ES)
(74) Representative: ABG Patentes, S.L.

(57) **Abstract**

The present invention relates to a method for the prognosis of non-small cell lung cancer (NSCLC) based on the expression levels of BRCA1 and XPG genes. In this sense, the inventors of the present invention have discovered that high expression levels of BRCA1 gene and low expression levels of XPG gene are correlated with a poor prognosis of a subject suffering from NSCLC. Moreover, the invention also relates to a method for designing an individual chemotherapy in a subject suffering from said disease.

SEQUENCE LISTING

<110>  PANGAEA BIOTECH, S.A.

<120>  Method for the prognosis of non-small cell lung cancer

<130>  P3665EP00

<160>  15

<170>  PatentIn version 3.3

<210>  1
<211>  18
<212>  DNA
<213>  Artificial

<220>
<223>  PCR β-actin forward primer

<400>  1
tgagcgcggc tacagctt
18


<210>  2
<211>  22
<212>  DNA
<213>  Artificial

<220>
<223>  PCR β-actin reverse primer

<400>  2
tccttaatgt cacgcacgat tt
22


<210>  3
<211>  18
<212>  DNA
<213>  Artificial

<220>
<223>  5' labeled fluorescent reporter dye (6FAM) probe for β-actin

<400>  3
accaccacgg ccgagcgg
18


<210>  4
<211>  26
<212>  DNA
<213>  Artificial

<220>
<223>  PCR BRCA1 forward primer

<400>  4

ggctatcctc tcagagtgac atttta
26


<210> 5
<211> 25
<212> DNA
<213> Artificial

<220>
<223> PCR BRCA1 reverse primer

<400> 5
gctttatcag gttatgttgc atggt
25


<210> 6
<211> 15
<212> DNA
<213> Artificial

<220>
<223> 5' labeled fluorescent reporter dye (6FAM) probe for BRCA1

<400> 6
ccactcagca gaggg
15


<210> 7
<211> 21
<212> DNA
<213> Artificial

<220>
<223> PCR ERCC1 forward primer

<400> 7
gggaatttgg cgacgtaatt c
21


<210> 8
<211> 18
<212> DNA
<213> Artificial

<220>
<223> PCR ERCC1 reverse primer

<400> 8
gcggaggctg aggaacag
18


<210> 9
<211> 25
<212> DNA
<213> Artificial

<220>
<223>  5' labeled fluorescent reporter dye (FAM) probe for ERCC1

<400>  9
cacaggtgct ctggcccagc acata
25


<210>  10
<211>  25
<212>  DNA
<213>  Artificial

<220>
<223>  PCR RRM1 forward primer

<400>  10
actaagcacc ctgactatgc tatcc
25


<210>  11
<211>  25
<212>  DNA
<213>  Artificial

<220>
<223>  PCR RRM1 reverse primer

<400>  11
cttccatcac atcactgaac acttt
25


<210>  12
<211>  28
<212>  DNA
<213>  Artificial

<220>
<223>  5' labeled fluorescent reporter dye (TAMRA) probe for RRM1

<400>  12
cagccaggat cgctgtctct aacttgca
28


<210>  13
<211>  20
<212>  DNA
<213>  Artificial

<220>
<223>  PCR XPG forward primer

<400>  13
gaagcgctgg aagggaagat
20

<210>  14
<211>  26
<212>  DNA
<213>  Artificial

<220>
<223>  PCR XPG reverse primer

<400>  14
gactccttta agtgcttggt ttaacc
26


<210>  15
<211>  21
<212>  DNA
<213>  Artificial

<220>
<223>  5' labeled fluorescent reporter dye (FAM) probe for XPG

<400>  15
ctggctgttg atattagcat t
21

SEQUENCE LISTING

<110>  PANGAEA BIOTECH, S.A.

<120>  Method for the prognosis of non-small cell lung cancer

<130>  P3665EP00

<160>  15

<170>  PatentIn version 3.3

<210>  1
<211>  18
<212>  DNA
<213>  Artificial

<220>
<223>  PCR ?-actin forward primer

<400>  1
tgagcgcggc tacagctt                                                    18

<210>  2
<211>  22
<212>  DNA
<213>  Artificial

<220>
<223>  PCR ?-actin reverse primer

<400>  2
tccttaatgt cacgcacgat tt                                               22

<210>  3
<211>  18
<212>  DNA
<213>  Artificial

<220>
<223>  5' labeled fluorescent reporter dye (6FAM) probe for ?-actin

<400>  3
accaccacgg ccgagcgg                                                    18

<210>  4
<211>  26
<212>  DNA
<213>  Artificial

<220>
<223>  PCR BRCA1 forward primer

<400>  4
ggctatcctc tcagagtgac atttta                                           26

<210>  5
<211>  25
<212>  DNA
<213>  Artificial

<220>
<223>  PCR BRCA1 reverse primer

<400>  5
gctttatcag gttatgttgc atggt                                          25


<210>  6
<211>  15
<212>  DNA
<213>  Artificial

<220>
<223>  5' labeled fluorescent reporter dye (6FAM) probe for BRCA1

<400>  6
ccactcagca gaggg                                                     15


<210>  7
<211>  21
<212>  DNA
<213>  Artificial

<220>
<223>  PCR ERCC1 forward primer

<400>  7
gggaatttgg cgacgtaatt c                                              21


<210>  8
<211>  18
<212>  DNA
<213>  Artificial

<220>
<223>  PCR ERCC1  reverse primer

<400>  8
gcggaggctg aggaacag                                                  18


<210>  9
<211>  25
<212>  DNA
<213>  Artificial

<220>
<223>  5' labeled fluorescent reporter dye (FAM) probe for ERCC1

<400>  9
cacaggtgct ctggcccagc acata                                          25


<210>  10
<211>  25
<212>  DNA
<213>  Artificial

<220>
<223>  PCR RRM1 forward primer

<400>  10
actaagcacc ctgactatgc tatcc                                          25


<210>  11
<211>  25
<212>  DNA
<213>  Artificial

<220>
<223>  PCR RRM1 reverse primer

<400>  11
cttccatcac atcactgaac acttt                                          25


<210>  12
<211>  28
<212>  DNA
<213>  Artificial

<220>
<223>  5' labeled fluorescent reporter dye (TAMRA) probe for RRM1

<400>  12
cagccaggat cgctgtctct aacttgca                                       28


<210>  13
<211>  20
<212>  DNA
<213>  Artificial

<220>
<223>  PCR XPG forward primer

<400>  13
gaagcgctgg aagggaagat                                                20


<210>  14
<211>  26
<212>  DNA
<213>  Artificial

<220>
<223>  PCR XPG reverse primer

<400>  14
gactccttta agtgcttggt ttaacc                                         26


<210>  15
<211>  21
<212>  DNA
<213>  Artificial

<220>
<223>  5' labeled fluorescent reporter dye (FAM) probe for XPG

<400>  15
ctggctgttg atattagcat t                                              21

## Description

### TECHNICAL FIELD OF THE INVENTION

The present invention relates to a method for the prognosis of non-small cell lung cancer and to a method for designing an individual chemotherapy in a subject suffering from said disease.

### BACKGROUND

Non-small-cell lung cancer (NSCLC) accounts for approximately 80% of all lung cancers, with 1.2 million new cases worldwide each year. NSCLC resulted in more than one million deaths worldwide in 2001 and is the leading cause of cancer-related mortality in both men and women (31% and 25%, respectively). The prognosis of advanced NSCLC is dismal. A recent Eastern Cooperative Oncology Group trial of 1155 patients showed no differences among the chemotherapies used: cisplatin/paclitaxel, cisplatin/gemcitabine, cisplatin/docetaxel and carboplatin/paclitaxel. Overall median time to progression was 3.6 months, and median survival was 7.9 months.

At diagnosis, patients with NSCLC can be divided into three groups that reflect both the extent of the disease and the treatment approach:
■ The first group of patients has tumors that are surgically resectable (generally stage I, stage II, and selected stage III tumors). This group has the best prognosis.
■ The second group includes patients with either locally (T3-T4) and/or regionally (N2-N3) advanced lung cancer. Patients with unresectable or N2-N3 disease are treated with radiation therapy in combination with chemotherapy. Selected patients with T3 or N2 disease can be treated effectively with surgical resection and either preoperative or postoperative chemotherapy or chemoradiation therapy.
■ The final group includes patients with distant metastases (M1). This group can be treated with pallieative radiation therapy or chemotherapy.

The overall five-year survival of patients with NSCLC has remained at less than 15% for the past 20 years. Stage grouping of TNM subsets (T=primary tumor; N=regional lymph nodes; M=distant metastases) permits the identification of patient groups with similar prognosis and treatment options. Five-year survival is around 25% for pathologic stage IIB (T1-2N1M0, T3N0M0), 13% for stage IIIA (T3N1M0, T1-2-3N2M0), and a low 7% for stage IIIB (T4N0-1-2M0).

Multiple studies have attempted to identify prognostic determinants after surgery and have yielded conflicting evidence as to the prognostic importance of a variety of clinicopathologic factors. Factors that have been observed to correlate with adverse prognosis include the following:
- Presence of pulmonary symptoms.
- Large tumor size (>3 cm).
- Non-squamous histology.
- Metastases to multiple lymph nodes within a TNM-defined nodal station.
- Vascular invasión.
- Increased numbers of tumor blood vessels in the tumor specimen.

Similarly, conflicting results regarding the prognostic importance of aberrant expression of a number of proteins within lung cancers have been reported. One of the proteins that is known to show certain prognostic value in NSCLC is the G member of the XP family of proteins, involved in the nucleotide-excision repair (NER) pathway. The NER pathway consists of ca. 30 proteins involved in DNA damage recognition, incision, DNA ligation and resynthesis. The NER pathway consists of several genes termed Xeroderma Pigmentosum (XP) or excision repair cross complementing (ERCC) where *XPA, ERCC1*, *ERCC2*/*XPD, ERCC4*/*XPF* and *ERCC5*/*XPG* are central. The human gene responsible for XP group G was identified as ERCC5/XPG (GenBank accession no. NM_000123). The XPG gene maps to chromosome 13q32-33, encodes for a structure-specific endonuclease that cleaves damaged DNA at 5 nucleotides 3' to the site of the lesion and is also required non-enzymatically for subsequent 5' incision by the XPF/ERCC1 heterodimer during the NER process. Several research groups have disclosed the relationship between the expression levels of XPG gene and the risk of lung cancer. For example, Cheng et al. (Carcinogenesis, 2000 vol. 21(8): 1527-1530) disclose the relationship between reduced expression levels of XPG gene and increased lung cancer risk. On the other hand, Zienolddiny et al.. (Carcinogenesis, 2006 vol. 27(3):560-567) disclose the association between *ERCC5*/*XPG* polymorphisms (His1104Asp) and the reduced risk of squamous cell carcinoma, being biologically plausible since XPG protein plays an important role in NER.

Another gene involved in DNA repair which has been shown to be suitable as prognostic marker for different types of cancer is the BRCA1. BRCA1 is implicated in transcription-coupled nucleotide excision repair (TC-NER), and modulation of its expression leads to modification of TC-NER and hence to radio- and chemoresistance. Upregulation of BRCA1 expression led to increased cisplatin resistance in the SKOV-3 human ovarian cancer cell line (Husain A, et al. Cancer Res. 1998 vol. 58 (6): 1120-3) and restoration of BRCA1 in the BRCA1-negative HCC1937 human breast cancer cell line restored radioresistance. BRCA1 is also involved in homologous recombination repair (HRR) and non-homologous end joining in response to DNA damage. In addition, it is a component of a large DNA repair complex termed the BRCA1-associated genome surveillance complex, which contains a number of mismatch repair proteins, indicating a potential role for BRCA1 in mismatch repair. BRCA1 may also be a regulator of mitotic spindle assembly, as BRCA1 and β-tubulin colocalize to the microtubules of the mitotic spindle and to the centrosomes. Finally, enhanced BRCA1 expression has been linked to apoptosis through the c-Jun N-terminal kinase pathway, which is activated by cisplatin-induced DNA damage; inhibition of this pathway increased cisplatin sensitivity in cell lines.

Altered BRCA1 mRNA expression has been observed in both sporadic and hereditary breast cancers (Kennedy RD, et al. 2002 Lancet, vol. 360, 1007-1014). These patients can respond to DNA damage-based chemotherapy but not to antimicrotubule drugs. In addition, DNA damage-based chemotherapy confers a significant survival advantage to BRCA1 mutation carriers compared to non-mutation carriers. Also ovarian cancer patients with low levels of BRCA1 mRNA have improved survival following platinum-based chemotherapy compared to patients with high levels of BRCA1 mRNA (Quinn et al., 2007Clin Cancer Res. vol. 13(24):7413-20).

Moreover, the BRCA1 gene has also been shown to be a prognostic marker of NSCLC. For instance, the US patent application US2006/0094021 and Taron et al. 2004 (Human Molecular Genetics, 2004 vol. 13(20): 2443-2449) disclose that BRCA1 mRNA expression levels is a good marker of differential sensitivity to chemotherapy in NSCLC, providing an important tool for customizing NSCLC chemotherapy in order to improve survival in this very common and fatal disease. On the other hand, Rosell *et al.* (PLoS ONE, 2007, 2:el 129) discloses that overexpression of BRCA1 mRNA is strongly associated with poor survival in NSCLC patients.

Thus, there is a need in the art for further prognosis markers for predicting the clinical outcome of a patient suffering from NSCLC.

### SUMMARY OF THE INVENTION

In one aspect, the present invention relates to an *in vitro* method for predicting the clinical outcome of a subject suffering from non-small cell lung cancer (NSCLC) comprising
(a) determining the expression levels of BRCA1 gene and XPG gene in a sample from the subject and
(b) comparing said expression levels of BRCA1 gene and XPG gene with standard reference values for each gene,
wherein high expression levels of BRCA1 gene and low expression levels of XPG gene with respect to said reference values, is indicative of a poor clinical outcome of the subject.

In another aspect, the invention relates to an *in vitro* method for designing an individual therapy for a subject suffering from NSCLC comprising
(a) determining the expression levels of BRCA1 gene and XPG gene in a sample from the subject and
(b) comparing said expression levels of BRCA1 gene and XPG gene with standard reference values for each gene,
wherein high expression levels of BRCA1 gene and low expression levels of XPG gene with respect to said reference values, is indicative of that said subject is a candidate for an adjuvant therapy.

In another aspect, the invention relates to a kit comprising agents capable of specifically detecting the expression levels of BRCA1 gene and/or XPG.

Uses of said kit are additional aspects of the present patent application. Thus, in another aspect, the invention relates to the use of the kit of the invention for predicting the clinical outcome of a subject suffering from NSCLC, wherein if said agents detect a high expression levels of BRCA1 gene and low expression levels of XPG gene, with respect to reference values, then clinical outcome of the subject is poor.

Finally, in another aspect, the invention relates to the use of the kit of the invention for designing an individual chemotherapy of a subject suffering from NSCLC, wherein if said agents detect a high expression levels of BRCA 1 gene and low mRNA expression levels of XPG gene with respect to reference values, then the subject is a candidate for an adjuvant therapy.

### BRIEF DESCRIPTION OF THE FIGURES

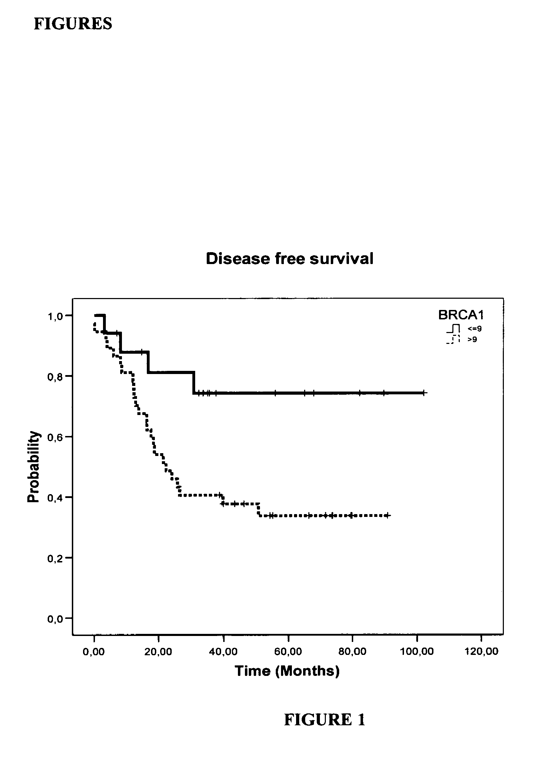

**Figure 1** is a graphic showing the probability of disease free survival as a function of BRCA1 gene expression levels.

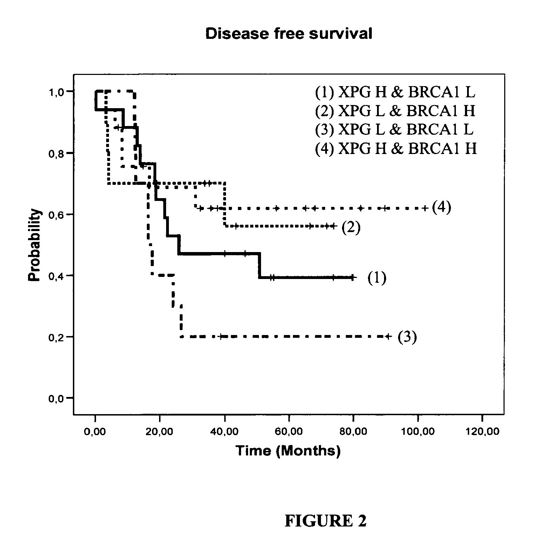

**Figure 2** is a graphic showing the probability of disease free survival as a function of BRCA1 gene and XPG gene expression levels. H: high expression levels; L: low expression levels.

### DETAILED DESCRIPTION OF THE INVENTION

Multiple studies have attempted to identify NSCLC prognostic determinants after surgery and have yielded conflicting evidence as to the prognostic importance of a variety of clinicopathologic factors. Surprisingly, the inventors of the present invention have now discovered that it is possible to establish the prognosis of NSCLC in a subject by taking into account simultaneously the expression levels of BRCA1 and XPG genes. In this sense, high expression levels of BRCA1 gene and low expression levels of XPG gene are correlated with a poor prognosis of a subject suffering from NSCLC.

Thus, in a first aspect, the present invention relates to an *in vitro* method for predicting the clinical outcome of a subject suffering from NSCLC comprising
(a) determining the expression levels of BRCA1 gene and XPG gene in a sample from the subject and
(b) comparing said expression levels of BRCA1 gene and XPG gene with standard reference values for each gene,
wherein high expression levels of BRCA1 gene and low expression levels of XPG gene with respect to said reference values, is indicative of a poor clinical outcome of the subject.

In the present invention "clinical outcome" is understood as the expected course of a disease. It denotes the doctor's prediction of how a subject's disease will progress, and whether there is chance of recovery o recurrence.

The prediction of the clinical outcome can be done by using any endpoint measurements used in oncology and known to the skilled practitioner. Useful endpoint parameters to describe the evolution of a disease include:
■ disease-free progression which, as used herein, describes the proportion of subjects in complete remission who have had no recurrence of disease during the time period under study;
■ objective response, which, as used in the present invention, describes the proportion of treated people in whom a complete or partial response is observed;
■ tumor control, which, as used in the present invention, relates to the proportion of treated people in whom complete response, partial response, minor response or stable disease ≥ 6 months is observed.
■ progression free survival which, as used herein, is defined as the time from start of treatment to the first measurement of cancer growth.
■ six-month progression free survival or "PFS6" rate which, as used herein, relates to the percentage of people wherein free of progression in the first six months after the initiation of the therapy and
■ median survival which, as used herein, relates to the time at which half of the subjects enrolled in the study are still alive.

In a particular embodiment, the clinical outcome is measured as disease-free-survival.

In the present invention "disease-free survival" (DFS) is understood as the length of time after treatment for a specific disease during which a subject survives with no sign of the disease.

In the context of the present invention, the disease is NSCLC. Thus, the *in vitro* method of the invention is applicable to a subject suffering from NSCLC. In a particular embodiment, the NSCLC is selected from squamous cell carcinoma of the lung, large cell carcinoma of the lung, and adenocarcinoma of the lung. Furthermore, the present method can also be applicable to a subject suffering from any stage of NSCLC (stages 0, IA, IB, IIa, IIb, IIIa, IIIb o IV). However, in a particular embodiment, the NSCLC is early NSCLC.

In the present invention "early NSCLC" refers to cancer that has not spread outside the lung to distant sites in the body.

In performing the method of the present invention, a sample is obtained from the subject. The term "sample" as used herein, relates to any sample which can be obtained from the subject. The present method can be applied to any type of biological sample from a subject, such as a biopsy sample, tissue (e.g. pulmonary tissue), cell or fluid (serum, saliva, semen, sputum, cerebral spinal fluid (CSF), tears, mucus, sweat, milk, brain extracts and the like).

In a particular embodiment, said sample is a tumour tissue sample or portion thereof. Preferably, said tumor tissue sample is a pulmonary tumor tissue sample from a subject suffering from NSCLC who is receiving or has previously received anti-cancer treatmen. Said sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Tumour cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze.

As the person skilled in the art understands, the expression levels of BRCA1 gene and XPG gene can be measured by determining the mRNA expression levels of said genes or by determining the protein levels encoded by said genes, i.e. BRCA1 protein and XPG protein.

Thus, in a particular embodiment, the expression levels of BRCA1 gene and XPG gene are measured by determining mRNA expression levels of said genes.

In order to measure the mRNA levels of BRCA1 and XPG genes, the biological sample may be treated to physically or mechanically disrupt tissue or cell structure, to release intracellular components into an aqueous or organic solution to prepare nucleic acids for further analysis. The nucleic acids are extracted from the sample by procedures known to the skilled person using commercially available reagents. RNA is then extracted from frozen or fresh samples by any of the methods typical in the art [Sambrook, Fischer and Maniatis, Molecular Cloning, a laboratory manual, (2nd ed.), Cold Spring Harbor Laboratory Press, New York, (1989)]. Preferably, care is taken to avoid degradation of the RNA during the extraction process.

The expression level can be determined using mRNA obtained from a formalin-fixed, paraffin-embedded tissue sample coming from a subject as defined above. In this case, the tissue sample is first deparaffinized. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample.

While all techniques of gene expression profiling (Real Time-PCR, SAGE, or TaqMan®) are suitable for use in performing the foregoing aspects of the invention, the mRNA expression levels are often determined by reverse transcription polymerase chain reaction (RT-PCR). The detection can be carried out in individual samples or in tissue microarrays.

In a particular embodiment, the mRNA expression levels of BRCA1 and XPG genes are determined by quantitative PCR, preferably, Real-Time PCR.

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts in tumor cells with respect to non-tumorigenic cells. Preferably, the control RNA are mRNA derived from housekeeping genes and which code for proteins which are constituvely expressed and carry out essential cellular functions. Examples of housekeeping genes for use in the present invention include β-2-microglobulin, ubiquitin, 18-S ribosomal protein, cyclophilin, GAPDH and β-actin. In a particular embodiment, the control RNA is β-actin mRNA. In one embodiment, relative gene expression quantification is calculated according to the comparative Ct method using β-actin as an endogenous control and commercial RNA controls as calibrators. Final results, are determined according to the formula 2-(ΔCt sample-ΔCt calibrator), where ΔCT values of the calibrator and sample are determined by subtracting the CT value of the target gene from the value of the β-actin gene

The determination of the level of expression of the BRCA1 gene and XPG gene needs to be correlated with reference values which correspond to the median value of expression levels of BRCA1 gene and XPG gene measured in a collection of tumor tissue in biopsy samples from NSCLC subjects obtained previous to the adjuvant chemotherapeutic treatment. Once this median value is established, the level of this marker expressed in tumor tissues from the subject can be compared with this median value, and thus be assigned a level of "low," "normal" or "high" expression. The collection of samples from which the reference level is derived will preferably be constituted from subjects suffering from the same type of cancer, i.e. NSCLC.

For example, the one described in the examples which is statistically representative was constituted with 54 tumor samples from subjects suffereing NSCLC, although it can contain a different number of samples. The use of a reference value used for determining whether the expression of a gene sample is "high" or "low" corresponds to the median value of expression levels of BRCA1 gene and XPG gene measured in a RNA sample obtained by pooling equal amounts of RNA from each of the tumour samples obtained by biopsy from NSCLC subjects previous to the neoadjuvant chemotherapeutic treatment. Once this median value is established, the level of this marker expressed in tumor tissues from the subject can be compared with this median value, and thus be assigned a level of "high" or "low" expression. In a particular embodiment, an increase in expression above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "high" expression. In a particular embodiment, a decrease in expression below the reference value of at least 0.9-fold, 0.75-fold, 0.2-fold, 0.1-fold, 0.05-fold, 0.025-fold, 0.02-fold, 0.01-fold, 0.005-fold or even less compared with the reference value is considered as "low" expression.

Due to inter-subject variability (e.g. aspects relating to age, race, etc.) it is very difficult (if not practically impossible) to establish absolute reference values for BRCA1 gene and XPG gene. Thus, in this case, the reference values for "high" or "low" expression of BRCA1 and XPG genes are determined by calculating percentiles by conventional means involving the testing of a group of samples isolated from normal subjects (i.e. people with no diagnosis of NSCLC) for the expression levels of the BRCA1 and XPG genes. For example, the "high" levels can then be assigned, preferably, to samples wherein expression levels for the BRCA1 and XPG genes are equal to or in excels of percentile 50 in the normal population, including, for example, expression levels equal to or in excess to percentile 60 in the normal population, equal to or in excess to percentile 70 in the normal population, equal to or in excess to percentile 80 in the normal population, equal to or in excess to percentile 90 in the normal population, and equal to or in excess to percentile 95 in the normal population. In another embodiment, the expression levels are assigned as "high" or "low" according to their values with respect to the median, wherein the median is the value which separates the higher half of a sample from the lower half. By using the median as cut off value for selecting those patients with high and low expression levels, at most half the population have values less than the median and at most half have values greater than the median.

In another particular embodiment, the expression levels of BRCA1 gene and XPG gene are measured by determining the protein levels encoded by said genes, i.e. BRCA1 and XPG proteins.

Practically any conventional method can be used within the context of the present invention to quantify the levels of BRCA1 and XPG proteins. By way of non-limiting example, the levels of said proteins can be quantified by means of conventional methods, for example, using antibodies with a capacity to specifically bind to BRCA1 protein and/or XPG protein (or to fragments thereof containing antigenic determinants) and subsequent quantification of the resulting antibody-antigen complexes.

The antibodies to be employed in these assays can be, for example, polyclonal sera, hybridoma supernatants or monoclonal antibodies, antibody fragments, Fv, Fab, Fab' y F(ab')2, ScFv, diabodies, triabodies, tetrabodies and humanised antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc. There are a wide variety of well-known assays that can be used in the present invention, which use non-labeled antibodies (primary antibody) and labeled antibodies (secondary antibodies); among these techniques are included Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as dipsticks. Other ways of detecting and quantifying the BRCA1 protein and XPG protein include techniques of affinity chromatography, binding-ligand assays, etc.

On the other hand, the determination of BRCA1 and XPG proteins expression levels can be carried out by constructing a tissue microarray (TMA) containing the subject samples assembled, and determining the expression levels of BRCA1 protein and XPG protein by immunohistochemistry techniques. Immunostaining intensity can be evaluated by two different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumoral cells and the specific cut-off for each marker. As a general criterion, the cutoffs were selected in order to facilitate reproducibility, and when possible, to translate biological events.

The findings of the inventors allow the development of personalised therapies for subjects suffering from NSCLC since high expression levels of BRCA1 gene and low expression levels of XPG gene correlate with a poor prognosis of the subject and, therefore, the subject will be a candidate for receiving an adjuvant therapy.

Thus, in another aspect, the invention relates to an *in vitro* method for designing an individual therapy for a subject suffering from NSCLC comprising
(a) determining the expression levels of BRCA1 gene and XPG gene in a sample from the subj ect and
(b) comparing said expression levels of BRCA1 gene and XPG gene with standard reference values for each gene,
wherein high expression levels of BRCA1 gene and low expression levels of XPG gene with respect to said reference values, is indicative of that said subject is a candidate for an adjuvant therapy.

In a particular embodiment of the method for designing an individual therapy for a subject suffering from NSCLC, the NSCLC is selected from squamous cell carcinoma of the lung, large cell carcinoma of the lung, and adenocarcinoma of the lung. Additionally, the present method can also be used for designing an individual therapy for a subject suffering from any stage of NSCLC (stages 0, IA, IB, IIa, IIb, IIIa, IIIb o IV). However, in a particular embodiment, the NSCLC is early NSCLC. The meaning of "early NSCLC" has been previously defined.

Any kind of sample can be used for carrying out the present method, i.e. a biopsy sample, tissue (e.g. pulmonary tissue), cell or fluid (serum, saliva, semen, sputum, cerebral spinal fluid (CSF), tears, mucus, sweat, milk, brain extracts and the like).. However, in a particular embodiment, the sample from the subject is a tumor tissue sample or portion thereof. Preferably, said tumor tissue sample is a pulmonary tumor tissue sample from a subject suffering from NSCLC who are receiving or have previously received anti-cancer treatment, in particular, anti-NSCLC treatment.

The present method requires determining the expression levels of the BRCA1 gene and the XPG gene. In a particular embodiment, the determination of the expression levels of the BRCA1 gene and the XPG gene can be carried out by measuring respectively the expression levels of the mRNA encoded by the BRCA1 gene and the XPG gene. More details about the different techniques for determining the mRNA expression levels of the BRCA1 gene and the XPG gene can be found in previous paragraphs from the present description and in Sambrook *et al*. 1989 (cited *at supra*). In another particular embodiment, the mRNA expression levels are determined by quantitative PCR, preferably, Real-Time PCR.

In another particular embodiment, the expression levels of BRCA1 gene and XPG gene are measured by determining the protein levels encoded by said genes, i.e. BRCA1 protein and XPG protein. All the techniques and conventional methods for determining the protein levels of BRCA1 protein and XPG protein have been already cited previously and can be applicable to the present method.

In the present invention "adjuvant therapy" is understood as the treatment given after the primary treatment to kill any cancer cells that may have spread, even if the spread cannot be detected by radiologic or laboratory tests, in order to increase the chances of a cure. As it is known for the skilled man, the primary treatment of choice for subject suffering from NSCLC is surgical resection. For inoperable, non-metastatic disease, radiotherapy to the thorax is likely to be considered. Adjuvant therapy may include chemotherapy, radiation therapy, hormone therapy, or biological therapy.

In a particular embodiment, the adjuvant therapy is selected from regional treatment, systemic treatment and combinations thereof.

In the present invention, "regional treatment" is understood as the treatment that is targeted to an area of the body surrounding the tumor.

In the present invention, "systemic treatment" is understood as the treatment that reaches cells all over the body by traveling through the bloodstream.

In another particular embodiment, the regional treatment is radiotherapy.

In another particular embodiment, the systemic treatment is selected from chemotherapy, immunotherapy and combinations thereof.

In another particular embodiment, the chemotherapy comprises cisplatin, carboplatin, paclitaxel (Taxol), docetaxel (Taxotere), topotecan, irinotecan, vinorelbine, gemcitabine or combinations thereof..

Additionally, the present invention relates to a kit useful to put into practice the methodology herein described. The kit contains the necessary reagents for the detection of the expression levels of BRCA1 gene and/or XPG gene.

Thus, in another aspect, the invention relates to a kit, hereinafter kit of the invention, comprising agents capable of specifically detecting the expression levels of BRCA1 gene and/or XPG gene.

In the present invention, a "kit" is understood as a product containing the different reagents for the detection of expression levels of BRCA1 gene and/or XPG gene.

Another component which can be present in the kit is a packing which allows maintaining the reagents within determined limits. Suitable materials for preparing such packings include glass, plastic (polyethylene, polypropylene, polycarbonate and the like), bottles, vials, paper, sachets and the like. The kit of the invention can additionally contain instructions for using the reagents in the method of the invention. Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain Internet websites providing said instructions.

In a particular embodiment, the reagents of the kit are capable of specifically detecting the levels of the mRNA encoded by the BRCA1 gene and by the XPG gene. In another embodiment, the reagents of the kit are capable of specifically detecting the levels of the BRCA1 protein and of the XPG protein.

Agents capable of specifically detecting the levels of the mRNA encoded by the BRCA1 gene and XPG gene are:
(i) oligonucleotide primers capable of specifically amplifying a fragment of BRCA1 gene or XPG gene nucleotide sequence; and

(j) oligonucleotide probes complementary to a fragment of BRCA1 gene or XPG gene nucleotide sequence.

As the skilled person understands, the oligonucleotide primers and probes of the kit of the invention can be use in all techniques of gene expression profiling (RT-PCR, SAGE, TaqMan, Real Time- PCR, etc.).

Agents capable of specifically detecting the expression levels of the BRCA1 protein and/or XPG protein are antibodies with a capacity to specifically bind to BRCA1 protein and/or XPG protein (or to fragments thereof containing antigenic determinants). Examples of the antibodies to be employed in the present invention have been previously cited. The antibodies of the kit of the invention can be used in conventional methods for detecting protein expression levels, such as Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips, protein microarrays including specific antibodies or assays based on colloidal precipitation in formats such as dipsticks, etc.

The different uses of the kit of the invention constitute additional aspects of the present invention.

Thus, in another aspect, the invention relates to the use of the kit of the invention for predicting the clinical outcome of a subject suffering from NSCLC, wherein if said agents detect high expression levels of BRCA1 gene and low expression levels of XPG gene, with respect to reference values, then the clinical outcome of the subject is poor.

Finally, in another aspect, the invention relates to the use of the kit of the invention for designing an individual chemotherapy for a subject suffering from NSCLC, wherein if said agents detect a high expression levels of BRCA1 gene and low expression levels of XPG gene with respect to reference values, then the subject is a candidate for an adjuvant therapy.

All the particular embodiments disclose for the methods of the present invention are applicable to the kit of the invention and uses thereof. The following examples are provided as merely illustrative and are not to be construed as limiting the scope of the invention.

### EXAMPLE

### XPG mRNA levels influence the effect of BRCA1 mRNA levels in prognosis of early non-small cell lung cancer (NSCLC)

### I. MATERIAL AND METHODS

### Patients

The patients were a median age of 66 years; 51 were male; Median disease-free survival (DFS) was 40 months for stage IA,was not reached for stage IB, and 25 months for stage II.

### Samples

The samples were selected from tumors of 54 resected NSCLC subjects. 42 of them suffer from squamous cell carcinomas and 12 from adenocarcinomas. The subjects were a median age of 66 years; 51 were male; Median disease-free survival (DFS) was 40 months for stage IA, not reached for stage IB, and 25 m for stage II.

### RNA extraction and Polymerase Chain Reaction

The BRCA1, ERCC1, RRM1 and XPG gene expression was measured as previously described by Specht K, et al. (2001) (Am. J. Pathol., 158, 419-429 and Krafft AE, et al. (1997) Mol. Diagn. 3, 217-230. After standard tissue sample deparaffinization using xylene and alcohols, samples were lysed in a Tris-chloride, EDTA, sodium dodecyl sulphate (SDS) and proteinase K containing buffer. RNA was then extracted with phenol-chloroform-isoamyl alcohol followed by precipitation with isopropanol in the presence of glycogen and sodium acetate. RNA was resuspended in RNA storage solution (Ambion Inc; Austin TX, USA) and treated with DNAse I to avoid DNA contamination. cDNA was synthesized using M-MLV retrotranscriptase enzyme. Template cDNA was added to Taqman Universal Master Mix (AB; Applied Biosystems, Foster City, CA, USA) in a 12.5-µl reaction with specific primers and probe for each gene. The primer and probe sets were designed using Primer Express 2.0 Software (AB). Quantification of gene expression was performed using the ABI Prism 7900HT Sequence Detection System (AB).

Primers and probe for BRCA1 mRNA expression analysis were designed according to the Ref Seq NM_007294 (http://www.ncbi.nlm.nih.gov/LocusLink). Forward primer is located in exon 8 (position 4292 bp to 4317 bp), reverse primer in exon 9 (position 4336 bp to 4360 bp), and probe in the exon 8/9 junction (position 4313 bp to 4333 bp). The PCR product size generated with these primers was 69 bp. The primers and 5'labeled fluorescent reporter dye (6FAM) probe were as follows: β-actin: forward 5' TGA GCG CGG CTA CAG CTT 3' [SEQ ID NO: 1], reverse 5' TCC TTA ATG TCA CGC ACG ATT T 3' [SEQ ID NO: 2], probe 5' ACC ACC ACG GCC GAG CGG 3' [SEQ ID NO:3]; BRCA1: forward 5'GGC TAT CCT CTC AGA GTG ACA TTT TA 3' [SEQ ID NO: 4], reverse 5' GCT TTA TCA GGT TAT GTT GCA TGG T 3' [SEQ ID NO: 5], probe 5' CCA CTC AGC AGA GGG 3' [SEQ ID NO: 6].

Primers and probe for ERCC1 mRNA expression analysis were designed according to the Ref Seq NT_011109 (http://www.ncbi.nlm.nih.gov/LocusLink). The primers and 5'labeled fluorescent reporter dye (FAM) probe were as follows: forward 5'GGG AAT TTG GCG ACG TAA TTC 3' [SEQ ID NO: 7]; reverse 5'GCG GAG GCT GAG GAA CAG 3'[SEQ ID NO: 8]; and probe 5' CAC AGG TGC TCT GGC CCA GCA CAT A 3' [SEQ ID NO: 9].

Primers and probe for RRM1 mRNA expression analysis were designed according to the Ref Seq NM_001033 (http://www.ncbi.nlm.nih.gov/LocusLink). The primers and 5'labeled fluorescent reporter dye (TAMRA) probe were as follows: forward 5'ACT AAG CAC CCT GAC TAT GCT ATC C 3' [SEQ ID NO: 10]; reverse 5'CTT CCA TCA CAT CAC TGA ACA CTT T 3' [SEQ ID NO: 11]; and probe 5'CAG CCA GGA TCG CTG TCT CTA ACT TGC A 3'[SEQ ID NO: 12].

Primers and probe for XPG mRNA expression analysis were designed according to the Ref Seq NM:000123 (http://www.ncbi.nlm.nih.gov/LocusLink). The primers and 5'labeled fluorescent reporter dye (FAM) probe were as follows: forward 5' GAA GCG CTG GAA GGG AAG AT 3' [SEQ ID NO: 13]; reverse 5'GAC TCC TTT AAG TGC TTG GTT TAA CC 3'[SEQ ID NO: 14]; and probe 5' CTG GCT GTT GAT ATT AGC ATT 3' [SEQ ID NO: 15].

Relative gene expression quantification was calculated according to the comparative Ct method using β-actin as an endogenous control and commercial RNA controls (Stratagene, La Jolla, CA) as calibrators. Final results, were determined as follows: 2-(ΔCt sample-ΔCt calibrator), where ΔC_{T} values of the calibrator and sample are determined by subtracting the C_{T} value of the target gene from the value of the β-actin gene. In all experiments, only triplicates with a standard deviation (SD) of the Ct value <0.20 were accepted. In addition, for each sample analyzed, a retrotranscriptase minus control was run in the same plate to assure lack of genomic DNA contamination.

### II. RESULTS

DFS was not reached in patients with low ERCC1 or RRM1 levels, while patients with high ERCC1 or RRM1 had a DFS of 26 months (P=ns). DFS was not reached in 18 patients with low BRCA1 mRNA levels, while 37 patients with high BRCA1 mRNA levels had a DFS of 22.4 months (P=0.02) [see table 1].

**Table 1**

| | N | Median | 95% CI | p |
|---|---|---|---|---|
| ≤9 | 18 | NR | - | 0.02 |
| >9 | 37 | 22.4 | 13.4-31.3 | |

In contrast, patients with high XPG had a 51-m DFS, while patients with low XPG had a 26 months DFS. When BRCA1 and XPG levels were considered jointly, DFS was not reached for patients with low levels of BRCA1 regardless of whether XPG was high (10 patients) or low (17 patients). However, for patients with high BRCA1 levels, DFS was shorter for 10 patients with low XPG (16.4 months) than for 17 patients with high XPG (26 months) (P=ns) [see Table 2].

**Table 2**

| | N | Median | 95% CI | p |
|---|---|---|---|---|
| XPG H & BRCA1 L | 10 | NR | - | 0.24 |
| XPG L & BRCA1 H | 10 | 16.4 | 14.3-18.4 | |
| XPG L & BRCA1 L | 17 | NR | - | |
| XPG H & BRCA1 H | 17 | 25.9 | 0-61.3 | |

High BRCA1 mRNA expression confers poor prognosis in early NSCLC and the combination of high BRCA1 plus low XPG expression still further increases the risk of shorter DFS. These findings could have important implications for the optimal use of adjuvant chemotherapy.

## Claims

1. An *in vitro* method for predicting the clinical outcome of a subject suffering from non-small cell lung cancer (NSCLC) comprising
(a) determining the expression levels of BRCA1 gene and XPG gene in a sample from the subject and
(b) comparing said expression levels of BRCA1 gene and XPG gene with standard reference values for each gene,
wherein high expression levels of BRCA1 gene and low expression levels of XPG gene with respect to said reference values, is indicative of a poor clinical outcome of the subject.

2. Method according to claim 1, wherein the clinical outcome is measured as disease-free-survival.

3. Method according to claims 1 or 2, wherein the sample is a tumor tissue sample.

4. Method according to any of claims 1 to 3, wherein the expression levels of BRCA1 gene and XPG gene are measured by determining mRNA expression levels of said genes.

5. Method according to claim 4, wherein the mRNA expression levels are determined by quantitative PCR, preferably, Real-Time PCR.

6. Method according to any of claims 1 to 3, wherein the expression levels of BRCA1 gene and XPG gene are measured by determining the levels of the proteins encoded by said genes.

7. Method according to any of claims 1 to 6, wherein the NSCLC is selected from squamous cell carcinoma of the lung, large cell carcinoma of the lung, and adenocarcinoma of the lung.

8. Method according to any of claims 1 to 7, wherein the NSCLC is early NSCLC.

9. An *in vitro* method for designing an individual therapy for a subject suffering from NSCLC comprising
(a) determining the expression levels of BRCA1 gene and XPG gene in a sample from the subject and
(b) comparing said expression levels of BRCA1 gene and XPG gene with standard reference values for each gene,
wherein high expression levels of BRCA1 gene and low expression levels of XPG gene with respect to said reference values, is indicative that said subject is a candidate for an adjuvant therapy.

10. Method according to claim 9, wherein the sample is a tumor tissue sample.

11. Method according to any of claims 9 or 10, wherein the expression levels of BRCA1 gene and XPG gene are measured by determining mRNA expression levels of said genes

12. Method according to claim 11, wherein the mRNA expression levels are determined by quantitative PCR, preferably, Real-Time PCR.

13. Method according to any of claims 9 or 10, wherein the expression levels of BRCA1 gene and XPG gene are measured by determining the levels of the proteins encoded by said genes.

14. Method according to any of claims 9 to 13, wherein the NSCLC is selected from squamous cell carcinoma of the lung, large cell carcinoma of the lung and adenocarcinoma of the lung.

15. Method according to anyone of claims 9 to 14, wherein the NSCLC is early NSCLC.

16. Method according to any of claims 9 to 15, wherein the adjuvant therapy is selected from regional treatment, systemic treatment and combinations thereof.

17. Method according to claim 16, wherein the regional treatment is radiotherapy.

18. Method according to claim 16, wherein the systemic treatment is selected from chemotherapy, immunotherapy, and combinations thereof.

19. Method according to claim 18, wherein the chemotherapy comprises cisplatin, carboplatin, paclitaxel (Taxol), docetaxel (Taxotere), topotecan, irinotecan, vinorelbine, gemcitabine or combinations thereof..

20. Kit comprising agents capable of specifically detecting the expression levels of BRCA1 gene and/or XPG gene.

21. Kit according to claim 20, wherein the agents of the kit are capable of specifically detecting the mRNA levels of BRCA1 gene and XPG gene or the levels of BRCA1 protein and/or XPG protein.

22. Use of a kit according to anyone of claims 20 and 21 for predicting the clinical outcome of a subject suffering from NSCLC, wherein if said agents detect a high expression levels of BRCA1 gene and low expression levels of XPG gene, with respect to reference values, then the clinical outcome of the subject is poor.

23. Use of a kit according to anyone of claims 20 and 21 for designing an individual chemotherapy for a subject suffering from NSCLC, wherein if said agents detect high expression levels of BRCA1 gene and low expression levels of XPG gene with respect to reference values, then the subject is a candidate for an adjuvant therapy.
